# FASCICULE DE BREVET EUROPEEN

(11) **EP 2 877 591 B1**
(45) Date de publication et mention de la délivrance du brevet: **10.01.2018**
(21) Numéro de dépôt: 13756613.9
(22) Date de dépôt: 29.07.2013
(51) Int. Cl.: G01N 33/569, C12Q 1/04, C12Q 1/10

(54) **PROCEDE DE DETECTION DE BACTERIES PRODUCTRICES DE CARBAPENEMASES DE TYPE OXA-048**
VERFAHREN ZUM NACHWEIS VON OXA-048-CARBAPENEMASE-PRODUZIERENDEN BAKTERIEN
METHOD OF DETECTING OXA-048 CARBAPENEMASE PRODUCING BACTERIA

(30) Priorité: 27.07.2012 FR 1257324
(43) Date de publication de la demande: 03.06.2015
(73) Titulaire: Biomérieux, 69280 Marcy l'Étoile (FR)
(72) Inventeur: DEVIGNE, Laurence, F-01150 Vaux en Bugey (FR); GHIRARDI, Sandrine, F-69290 Saint Genis les Ollières (FR); ZAMBARDI, Gilles, 38230 Tignieu Jameyzieu (FR)
(86) Numéro de dépôt international: PCT/FR2013/051819
(87) Numéro de publication internationale: WO 2014/016534

(56) Documents cités:
- D. GIRLICH ET AL: "Value of the Modified Hodge Test for Detection of Emerging Carbapenemases in Enterobacteriaceae", JOURNAL OF CLINICAL MICROBIOLOGY, vol. 50, no. 2, 23 novembre 2011 (2011-11-23), pages 477-479, XP055059204, ISSN: 0095-1137, DOI: 10.1128/JCM.05247-11
- G. VRIONI ET AL: "Comparative Evaluation of a Prototype Chromogenic Medium (ChromID CARBA) for Detecting Carbapenemase-Producing Enterobacteriaceae in Surveillance Rectal Swabs", JOURNAL OF CLINICAL MICROBIOLOGY, vol. 50, no. 6, 29 mars 2012 (2012-03-29), pages 1841-1846, XP055059207, ISSN: 0095-1137, DOI: 10.1128/JCM.06848-11
- YOURI GLUPCZYNSKI ET AL: "Rapid emergence and spread of OXA-48-producing carbapenem-resistant Enterobacteriaceae isolates in Belgian hospitals", INTERNATIONAL JOURNAL OF ANTIMICROBIAL AGENTS, ELSEVIER SCIENCE, AMSTERDAM, NL, vol. 39, no. 2, 10 octobre 2011 (2011-10-10), pages 168-172, XP028434352, ISSN: 0924-8579, DOI: 10.1016/J.IJANTIMICAG.2011.10.005 [extrait le 2011-11-03]
- P. NORDMANN ET AL: "Identification and screening of carbapenemase-producing Enterobacteriaceae", CLINICAL MICROBIOLOGY AND INFECTION, vol. 18, no. 5, 17 avril 2012 (2012-04-17) , pages 432-438, XP055059201, ISSN: 1198-743X, DOI: 10.1111/j.1469-0691.2012.03815.x
- L. POIREL ET AL: "OXA-48-like carbapenemases: the phantom menace", JOURNAL OF ANTIMICROBIAL CHEMOTHERAPY, vol. 67, no. 7, 11 avril 2012 (2012-04-11) , pages 1597-1606, XP055059209, ISSN: 0305-7453, DOI: 10.1093/jac/dks121
- D. M. LIVERMORE ET AL: "Temocillin revived", JOURNAL OF ANTIMICROBIAL CHEMOTHERAPY, vol. 63, no. 2, 19 novembre 2008 (2008-11-19), pages 243-245, XP055059203, ISSN: 0305-7453, DOI: 10.1093/jac/dkn511
- R. HARTL ET AL: "Temocillin and meropenem to discriminate resistance mechanisms leading to decreased carbapenem susceptibility with focus on OXA-48 in Enterobacteriaceae", CLINICAL MICROBIOLOGY AND INFECTION, vol. 19, no. 5, 9 février 2013 (2013-02-09), pages E230-E232, XP055059246, ISSN: 1198-743X, DOI: 10.1111/1469-0691.12146

## Description

La présente invention se rapporte au domaine de l'analyse microbiologique. Plus spécifiquement, elle porte sur un procédé de détection et/ou d'identification de bactéries productrices de carbapénémases de type OXA-48.

L'augmentation de la résistance aux antibiotiques de type beta-lactamines, tels les pénicillines et les céphalosporines, rend complexe le traitement des infections causées par des souches de bactéries Gram négatives. Ces antibiotiques sont alors remplacés par d'autres antimicrobiens à large spectre. Parmi ces antimicrobiens à large spectre, les carbapénèmes ont pris une place importante, en particulier pour traiter des patients hospitalisés. Les carbapénèmes sont actifs contre la majorité des bactéries aérobies Gram positives et Gram négatives ainsi que contre certaines bactéries anaérobies.

Cependant, de plus en plus de souches résistantes aux carbapénèmes apparaissent chez des patients hospitalisés. Et il est primordial de détecter rapidement les souches présentant une activité carbapénèmase pour instaurer le cas échéant une antibiothérapie permettant de traiter une infection de façon appropriée, et pour identifier les patients porteurs afin de diminuer le risque de propagation de ces souches dans les centres de soins..

La nomenclature des béta-lactamases, enzymes bactériennes de résistance aux beta-lactamines, n'est pas complètement standardisée. Elles sont soit classées en quatre classes moléculaires (A à D) sur la base de leur structure primaire (classification d'Ambler), soit en groupes fonctionnels sur la base des substrats ciblés et de leur résistance aux inhibiteurs (pour une revue, voir Bush and Jacoby, Antimicrobial Agents and Chemotherapy, 2010 ; 54 (3) : 969-976).

Les bactéries concernées par la résistance aux carbapénèmes sont, de façon non exhaustive, *Escherichia coli, Enterobacter cloacae, Enterobacter aerogenes, Citrobacter sp., Klebsiella pneumoniae, Klebsiella oxytoca, Pseudomonas aeruginosa, Providencia rettgeri, Pseudomonas putida, Stenotrophomonas maltophilia, Acinetobacter baumanii, Comamonas sp., Aeromonas sp., Morganella morganii, Enterococcus sp., Proteus mirabilis, Salmonella senftenberg, Serratia marcescens, Salmonella typhimurium* etc.

Ces bactéries peuvent produire différents types de beta-lactamases capables d'hydrolyser les carbapénèmes, ou carbapénémases. Les carbapénémases sont des beta-lactamases à très large spectre, capables d'inactiver presque la totalité des beta-lactamines. elles sont schématiquement réparties en trois classes selon la classification d'Ambler :
- la classe A, également appelée sérine-carbapénémase, se caractérise par une inhibition variable par l'acide clavulanique et les dérivés d'acide boronique. Le principal représentant est l'enzyme KPC ;
- la classe B, également appelée métallo-carbapénémase, se caractérise par une inhibition par des agents chélateurs de cations tels que l'EDTA. Les principaux représentants sont les enzymes NDM, VIM et IMP ;
- la classe D, qui correspond aux beta-lactamases de type OXA, parmi lesquelles figure le variant OXA-48 qui a la particularité de posséder une activité carbapénémase. D'autres variants dérivés par mutation ponctuelle d'OXA-48 existent, bien que moins fréquents, et conservent cette activité carbapénémase. On citera OXA-162, OXA-163, OXA-181, OXA-204 et OXA-232.

Les metallo-beta-lactamases (dont l'abréviation anglo-saxonne est MBLs) et les *Klebsiella pneumoniae* carbapenemase (KPC) sont prévalentes chez les entérobactéries plus particulièrement en Amérique du Nord, Amérique du Sud, Israël, Italie, Grèce, Inde, Chine et Pakistan. L'oxacillinase-48 (OXA-48) a été récemment isolée en Turquie, dans le bassin méditerranéen et en Europe de l'Ouest.

Les gènes de carbapénémases sont susceptibles d'être présents dans les chromosomes et/ou dans des plasmides. Du fait de cette présence sous forme de plasmides, ces résistances de type enzymatiques sont susceptibles de disséminer de façon très importante et présentent, en conséquence, un risque majeur en terme d'épidémiologie.

Pour détecter et/ou identifier des souches résistantes aux carbapénèmes, il est possible d'utiliser des techniques de biologie moléculaire qui sont très sensibles et présentent l'avantage de permettre une identification rapide des souches d'entérobactéries productrices de carbapénémases. Cependant, ces méthodes sont couteuses, complexes, et ne sont pas disponibles en routine dans la plupart des laboratoires.

Pour cribler, détecter et/ou identifier des entérobactéries productrices de carbapénémases (EPC), des méthodes basées sur la culture sont bien connues de l'homme de l'art. Elles reposent séquentiellement sur un isolement sur un milieu conventionnel de type Mac-Conkey agar ou en bouillon trypticase soja rendu sélectif par l'ajout d'un carbapénème ou après la réalisation d'un antibiogramme montrant une non sensibilité à un carbapénème. Le cas échéant, des disques imprégnés d'un carbapénème ou des bandelettes de type Etest® (bioMérieux) peuvent être utilisés.La confirmation de la présence de carbapénèmase par des tests complémentaires est nécessaire, tests parmi lesquels on citera : le test de Hodge modifié (CLSI M100-S22: Performance Standards for Antimicrobial Susceptibility Testing; Twenty-Second Informational Supplement. January 2012. Supplemental Table2A-S2), des tests de synergie par diffusion (ou disques combinés) en gélose, employant des inhibiteurs combinés au carbapénème, par exemple l'EDTA pour les carbapénèmases de classe B, l'acide phenyl-boronique pour la détection des carbapénèmases de classe A. La société Rosco propose une méthode multi-disques permettant de détecter des bactéries productrices de carbapénémases à partir de souches isolées (BioConnections KPC + MBL Confirm ID kit) et suggère d'ajouter un disque comprenant 30 µg de témocilline pour détecter la présence d'une enzyme OXA-48. Cependant, ce test montre que la témocilline n'inhibe pas l'ensemble des souches KPC, AmpC et MBL donc ne permet pas à elle seule une détection spécifique des souches OXA-48.

Ainsi, cet état de la technique ne permet pas la détection spécifique des EPC OXA-48 et nécessite un délai de réponse long, de trois jours. De plus, ces techniques sont mal adaptées à la recherche des CPE dans des prélèvements de selles ou rectaux qui sont utilisés pour la recherche de portage dans le cadre de la prévention des infections à bactéries multi-résistantes.

D'autres méthodes utilisent des milieux chromogènes commerciaux tels le milieu Brilliance CRE (Oxoid®), le milieu CHROMagar® KPC (CHROMagar™, Paris, France), le milieu équivalent Colorex KPC (Biomed Diagnostics Inc.), ou les milieux de la Demanderesse chromID® ESBL ou chromID® CARBA. Ce dernier milieu s'avère capable de détecter une souche OXA-48 de référence mais à condition que l'inoculum soit important (environ 10⁷ CFU/ml). Un autre milieu (Super Carba (Nordmann et al., 2012, J. Clin. Microbiol., *in press*)) a été décrit, qui permet une détection fiable des EPC productrices d'OXA-48, sans toutefois permettre leur identification, ni une détection spécifique car les bactéries produisant d'autres types de carbapénèmases ne sont pas inhibées. De plus, ce milieu présente l'inconvénient d'une très courte péremption (environ une semaine) qui limite fortement son utilisation en routine et son industrialisation.

Ainsi, aucune de ces méthodes n'est à la fois suffisamment sensible, spécifique et rapide pour la détection des souches OXA-48 et aucune solution d'amélioration n'a été proposée. La détection des souches OXA-48 présentant un réel intérêt clinique et épidémiologique, il reste à pallier les inconvénients des milieux existants dans ce domaine.

A cet égard, la présente invention porte sur un procédé de détection et/ou d'identification spécifique de bactéries productrices de carbapénémases de type OXA-48 dans un échantillon biologique, comprenant les étapes consistant à :
a) mettre en contact l'échantillon biologique, susceptible de contenir lesdites bactéries, avec un milieu réactionnel comprenant de la témocilline à une concentration supérieure ou égale à 150 mg/L, de préférence comprise entre 200 et 500 mg/ L, un agent chélatant des cations divalents de type EDTA, préférentiellement à une concentration comprise entre 1,0 et 2,5 mmol/L et un substrat chromogène permettant la détection d'une activité enzymatique spécifique,
b) incuber l'ensemble de façon à permettre la croissance des bactéries, et
c) détecter les souches correspondant aux bactéries productrices de carbapénémases de type OXA-48.

En effet, les travaux de la Demanderesse ont montré, de façon surprenante, que la témocilline à fortes concentrations, supérieures ou égales à 150 mg/L permet de discriminer spécifiquement les EPC de type OXA-48. De façon préférentielle, la témocilline est utilisée à une concentration comprise entre 150 et 500 mg/L. Ainsi, le procédé selon l'invention permet une détection sensible, spécifique et rapide (généralement en moins de 24 heures) des CPE de type OXA-48, tout en présentant l'avantage d'utiliser un milieu prêt-à-l'emploi et ayant une péremption longue permettant son industrialisation. Avantageusement, le procédé selon l'invention permet également une identification des souches.

La témocilline est un dérivé 6-alpha-methoxy de la ticarcilline qui est elle-même une pénicilline, parfois employée en combinaison avec l'acide clavulanique. Elle est décrite comme un traitement alternatif contre les entérobactéries multi-résistantes.

Des tests de susceptibilité *in vitro* ont été conduits par Livermore et al. (International Journal of Antimicrobial Agents, 2011 ; 37 : 415-419). Ils indiquent une concentration minimale inhibitrice (CMI) ≥ 256 mg/L, que l'homme du métier interprète comme signifiant que les bactéries testées poussent pour la dernière concentration testée en témocilline de 128 mg/L. Ces tests ont ainsi montré que 18/19 souches de type OXA-48 et 32/35 souches avec une métallo-carbapénémase étaient résistantes à la témocilline. Ceci dissuade l'homme du métier d'utiliser la témocilline dans un milieu visant à détecter spécifiquement les OXA-48.

Les définitions ci-après sont précisées afin de mieux comprendre l'invention.

Par échantillon biologique, on entend une petite partie ou petite quantité isolée d'une entité pour l'analyse. Ce peut être un échantillon clinique, humain ou animal, issu d'un prélèvement de liquide biologique, ou un échantillon alimentaire, issu de tout type d'aliment, ou un échantillon de l'environnement de production ou de transformation d'aliments. Cet échantillon peut être ainsi liquide ou solide. On peut citer d'une manière non limitative, un échantillon clinique de sang total, de sérum, de plasma, d'urines, de fécès, de liquide céphalo-rachidien, de prélèvements de nez, de gorge, de peau, de rectum, de plaie, un échantillon alimentaire d'eau, de boissons tels que le lait, un jus de fruits, de yaourt, de viande, d'oeufs, de légumes, de mayonnaise, de fromage, de poisson..., un échantillon alimentaire issu d'une alimentation destinée aux animaux, tel que notamment un échantillon issu de farines animales, un échantillon pour contrôle de surface ou d'eau. De façon préférentielle, selon la présente invention, l'échantillon est un échantillon clinique.

Le prélèvement peut être utilisé tel quel ou, préalablement à l'analyse, subir une préparation de type enrichissement, dilution, extraction, concentration, purification, selon des méthodes connues de l'homme du métier.

Par milieu réactionnel, on entend un milieu comprenant tous les éléments nécessaires à l'expression d'un métabolisme et/ou à la croissance de microorganismes. Le milieu réactionnel peut être solide, semi-solide ou liquide. Par milieu solide, on entend par exemple un milieu gélifié, ou gélosé. L'agar est l'agent gélifiant traditionnel en microbiologie pour la culture des microorganismes, mais il est possible d'utiliser de la gélatine, de l'agarose ou d'autres gélifiants naturels ou artificiels seuls ou en combinaison. Un certain nombre de préparations sont disponibles dans le commerce, comme par exemple l'agar Columbia, la gélose Trypcase-soja, la gélose Mac Conkey, la gélose Mueller Hinton ou plus généralement celles décrites dans le Handbook of Microbiological Media (CRC Press).

Le milieu réactionnel peut comprendre un ou plusieurs éléments en combinaison, tels que des acides aminés, des peptones, des hydrates de carbone, des nucléotides, des minéraux, des vitamines ... Le milieu peut comprendre également un colorant. A titre indicatif, on peut citer comme colorant le bleu d'Evans, du rouge neutre, du sang de mouton, du sang de cheval, un opacifiant tel que l'oxyde de Titane ou le kaolin, de la nitroaniline, du vert malachite, du vert brillant, un ou plusieurs indicateurs métaboliques, un ou plusieurs régulateurs métaboliques...

Le milieu réactionnel peut être un milieu de révélation ou un milieu de culture et de révélation. Dans le premier cas, la culture des microorganismes est effectuée avant ensemencement et, dans le deuxième cas, le milieu de détection et/ou d'identification constitue également le milieu de culture. L'identification signifie la classification des microorganismes dans une espèce ou un groupe d'intérêt.

L'homme du métier peut également utiliser une bi-boite, ou boite de type boite de Petri comprenant deux compartiments, permettant de comparer aisément deux milieux, comprenant différents substrats ou différents mélanges sélectifs, sur lesquels on aura déposé un même échantillon biologique.

Le milieu réactionnel peut comprendre un ou plusieurs agents sélectifs. Par agent sélectif, on entend tout composé susceptible d'empêcher ou de ralentir la croissance d'un microorganisme autre que le microorganisme cible. Sans être limitatif, une concentration comprise entre 0,01 mg/l et 5 g/l est particulièrement adaptée à la présente invention.

On peut citer comme agent sélectif, les antibiotiques, les antifongiques, les sels biliaires, le crystal violet, la fushine basique, le vert brillant, les tensioactifs tels le Tergitol™ ... Par antibiotique, on entend tout composé susceptible d'empêcher ou de ralentir la croissance d'une bactérie. Ils appartiennent notamment aux groupes des beta-lactamines, des glycopeptides, des aminosides, des polypeptides, des sulfamides, des quinolones. A titre indicatif, on peut citer notamment les antibiotiques carbénicilline, ticarcilline, témocilline, formidacilline, cefotaxime, cefsulodine, ceftazidime, cefoxitine, ceftriaxone, cefpodoxime, aztréonam, ertapénème, faropénème, doripénème, vancomycine, gentamicine, triméthoprime, tobramycine, moxalactam, fosfomycine, D-cyclosérine, polymixine, colistine, les quinolones telles que l'acide nalidixique.

Par antifongique, on entend tout composé susceptible d'empêcher ou de ralentir la croissance d'une levure ou d'une moisissure. A titre indicatif, on peut citer notamment l'amphotéricine B, le fluconazole, l'itraconazole, le voriconazole, la cycloheximide, la flucytosine.

Par substrat chromogène, on entend un substrat permettant la détection d'une activité enzymatique ou métabolique des microorganismes cibles grâce à un signal détectable directement ou indirectement. Pour une détection directe, ce substrat peut être lié à une partie faisant office de marqueur, fluorescent ou coloré (Orenga et al., 2009 ; J. Microbiol. Methods ; 79(2):139-55). Pour une détection indirecte, le milieu réactionnel selon l'invention peut comporter en sus un indicateur de pH, sensible à la variation de pH induite par la consommation du substrat et révélant le métabolisme des microorganismes cibles. Ledit indicateur de pH peut être un chromophore ou un fluorophore. On citera comme exemples de chromophores le bromocresol pourpre, le bleu de bromothymol, le rouge neutre, le bleu d'aniline, le bleu de bromocresol. Les fluorophores comprennent par exemple la 4-méthylumbelliferone, les dérivés de l'hydroxycoumarine ou les dérivés de la résorufine. Selon la présente invention, le substrat chromogène est préférentiellement choisi parmi les substrats à base d'Indoxyl (3-Indoxyl, 5-Bromo-3-indoxyl, 5-Iodo-3-indoxyl, 4-Chloro-3-indoxyl, 5-Bromo-4-chloro-3-indoxyl, 5-Bromo-6-chloro-3-indoxyl, 6-Bromo-3-indoxyl, 6-Chloro-3-indoxyl, 6-Fluoro-3-indoxyl, 5-Bromo-4-chloro-N-méthyl-3-indoxyl, N-Méthyl-3-indoxyl, Aldol™ ...) ; d'umbelliférone (4-Méthylumbelliférone, Cyclohexenoesculétine, ...) ; d'Alizarine ; de p-Naphtolbenzéine ; de Nitrophénol (ortho-Nitrophénol, para-Nitrophénol, ...) ; d'Hydroxyquinoline ; de Cathécol (Cathécol, Dihydroxyflavone, Hydroxyflavone, ...) ; de Résorufine; de Chlorophénol Red; de Fluorescéine; d'Aminophénol (para-Aminophénol, Dichloro-aminophénol, ...) ; de Naphtol (alpha-Naphtol, 2-Naphtol, Naphtol-ASBI, ...) ; d'Aminocoumarine (7-Amino-4-méthyl-coumarine, ...) ; de Naphtylamide; d'Acridine (Aminophényl-acridine...) ; d'Amino-phénoxazine (Amino-benzophénoxazinone, Amino-pentyl-resorufine, ...) ; d'Amino-styryl (Amino-styryl-quinolinium, Amino-styryl-lépidinium, ...).

A titre indicatif, les activités enzymatiques ciblées par les substrats chromogènes peuvent appartenir au groupe des hydrolases, préférentiellement aux groupes des osidases, des estérases ou des peptidases. Préférentiellement, les activités enzymatiques ciblées par les substrats chromogènes sont choisies parmi : la glucuronidase, la glucosidase, la galactosidase, la ribosidase, l'estérase, la sulfatase, la phospholipase, l'aminopeptidase et la désaminase.

A titre indicatif, les substrats utilisés pour la détection d'une activité beta-glucuronidase peuvent notamment être le 4-Méthylumbelliféryl-beta-glucuronide, le 5-Bromo-4-chloro-3-indolyl-beta-glucuronide, le 5-Bromo-6-chloro-3-indolyl-beta-glucuronide, le 6-Chloro-3-indolyl-beta-glucuronide, l'Aldol™-beta-glucuronide, l'Alizarine-beta-glucuronide, le Cyclohexenoesculetine-beta-glucuronide ou leurs sels.

Les substrats utilisés pour la détection d'une activité beta-galactosidase peuvent notamment être le 4-Méthylumbelliféryl-beta-galactoside, le 5-Bromo-4-chloro-3-indolyl-beta-galactoside, le 5-Bromo-6-chloro-3-indolyl-beta-galactoside, le 6-Chloro-3-indolyl-beta-galactoside, l'Aldol™-beta-galactoside, l'Alizarine-beta-galactoside, le Cyclohexenoesculetine-beta-galactoside ou leurs sels.

Les substrats utilisés pour la détection d'une activité alpha-galactosidase peuvent notamment être le 4-Méthylumbelliféryl-alpha-galactoside, le 5-Bromo-4-chloro-3-indolyl-alpha-galactoside, le 5-Bromo-6-chloro-3-indolyl-alpha-galactoside, le 6-Chloro-3-indolyl-alpha-galactoside ou leurs sels.

Les substrats utilisés pour la détection d'une activité beta-glucosidase peuvent notamment être le 4-Méthylumbelliféryl-beta-glucoside, le 5-Bromo-4-chloro-3-indolyl-beta-glucoside, le 5-Bromo-4-chloro-3-indolyl-N-méthyl-beta-glucoside, le 5-Bromo-6-chloro-3-indolyl-beta-glucoside, le 6-Chloro-3-indolyl-beta-glucoside, l'Aldol™-beta-glucoside, l'Alizarine-beta-glucoside, le Cyclohexenoesculetine-beta-glucoside, le Nitrophényl-beta-glucoside, le Dichloroaminophényl-glucoside ou leurs sels.

Les substrats utilisés pour la détection d'une activité alpha-glucosidase peuvent notamment être le 4-Méthylumbelliféryl-alpha-glucoside, le 5-Bromo-4-chloro-3-indolyl-alpha-glucoside, le 5-Bromo-4-chloro-3-indolyl-N-méthyl-alpha-glucoside, le 5-Bromo-6-chloro-3-indolyl-alpha-glucoside, le 6-Chloro-3-indolyl-alpha-glucoside, le Nitrophényl-alpha-glucoside ou leurs sels.

Les substrats utilisés pour la détection d'une activité ribosidase peuvent notamment être le 4-Méthylumbelliféryl-beta-riboside, le 5-Bromo-4-chloro-3-indolyl-beta-riboside, le 5-Bromo-6-chloro-3-indolyl-beta-riboside, le 6-Chloro-3-indolyl-beta-riboside, l'Alizarine-beta-riboside, le Nitrophényl-beta-riboside ou leurs sels.

A titre indicatif, les substrats utilisés pour la détection d'une activité estérase peuvent notamment être les esters d'acides gras linéaires saturés ou insaturés, ayant entre 6 et 14 carbones, préférentiellement entre 7 et 9 carbones et de 4-Méthylumbelliférone, 5-Bromo-4-chloro-3-indoxyl, 5-Bromo-6-chloro-3-indoxyl, 6-Chloro-3-indoxyl, 5-Bromo-3-indolyl ou d'Alizarine ou leurs sels. Préférentiellement, ils sont choisis parmi 4 Méthylumbellifyl octanoate, 5-Bromo-4 chloro-3-indoxyl octanoate, 5-Bromo-6-chloro-3-indoxyl octanoate, 6-Chloro-3-indoxyl octanoate, 5-Bromo-3-indolyl octanoate ou d'Alizarine octanoate.

Les substrats utilisés pour la détection d'une activité phospholipase peuvent notamment être le 4-Méthylumbelliféryl-phosphatidyl inositol, le 4-Méthylumbelliféryl-phosphatidyl choline, le 5-Bromo-4-chloro-3-indolyl-phosphatidyl inositol, 5-Bromo-4-chloro-3-indolyl-phosphatidyl choline, le Nitrophényl-phosphatidyl inositol, le Nitrophényl-phosphatidyl choline ou leurs sels.

Les substrats utilisés pour la détection d'une activité aminopeptidase peuvent notamment être la L-Alanyl-7-amido-4-méthylcoumarine, la L-Alanyl-dichloroamido-phényl, la L-Alanyl-7-amido-1-pentyl-phenoxazinone, la L-Alanyl-4-amidostyryl-quinaldinium, ou leurs sels.

Les substrats utilisés pour la détection d'une activité désaminase peuvent notamment être le L-Tryptophane, la L-Phénylalanine, la L-Tyrosine et la L-Histidine.

Les substrats utilisés pour la détection d'une activité sulfatase peuvent notamment être le 4-Méthylumbelliféryl-sulfate, le 5-Bromo-4-chloro-3-indoxyl-sulfate, le 5-Bromo-6-chloro-3-indoxyl-sulfate, le 3-Indoxyl-sulfate, le Phenolphtaléine-disulfate ou leurs sels. Préférentiellement, le substrat chromogène est choisi parmi : le 5-Bromo-4-chloro-3-indoxyl-beta-D-glucopyranoside (X-glucoside), le 5-Bromo-6 chloro-3-indoxyl-beta-D galactopyranoside (Magenta beta-Gal), le 6-Chloro-3-indoxyl-beta-D-glucuronide (Rose-beta-Gur), le 5-Bromo-4 chloro-3-indoxyl-N-méthyl-beta-D-glucopyranoside (GreenA-beta-Glu), le Méthyl-beta-D-glucopyranoside (methyl-beta-D-glucoside), le Lactose, le L-Tryptophane.

Le milieu réactionnel contient également au moins un agent chélatant des cations, de type EDTA, dans le but de complexer le zinc qui est un co-facteur des carbabapénémases de classe B, favorisant ainsi leur inhibition et pouvant ainsi restaurer l'activité d'une beta-lactamine telle que la témocilline. Avantageusement, la concentration en EDTA est comprise entre 1,0 et 2,5 mmol/L.

D'autres agents chélatants peuvent être cités à titre indicatif : l'acide dipicolinique, le 2-mercaptoethanol, des dérivés de la phénantroline.

Par incuber, on entend porter et maintenir entre 1 et 48 heures, préférentiellement entre 4 et 24 heures, plus préférentiellement entre 16 et 24 heures, à une température appropriée, généralement comprise entre 20 et 50°C, préférentiellement entre 30 et 40°C.

Par détecter, on entend déceler à l'oeil nu ou à l'aide d'un appareil optique ou numérique l'existence d'une croissance des bactéries cibles. Avantageusement, lorsque le milieu mis en oeuvre comprend un substrat chromogène, la détection peut également permettre une identification taxonomique des bactéries cibles. La détection se fait à l'oeil nu ou à l'aide d'un appareil optique ou numérique pour les substrats fluorescents et pour les substrats colorés. Par spécificité, on entend la capacité du procédé ou du milieu réactionnel à donner un résultat négatif lorsque la souche bactérienne recherchée n'est pas présente. En d'autres termes, selon la présente invention, une identification plus spécifique correspond à une réduction du nombre de faux positifs liés aux souches n'exprimant pas de carbapénémase de type OXA-48, sans prétendre inhiber l'ensemble de ces souches.

Par sensibilité, on entend la capacité à donner un résultat positif lorsque la souche bactérienne recherchée est présente dans l'échantillon.

Ainsi, la présente invention porte sur un procédé de détection et/ou d'identification spécifique de bactéries productrices de carbapénémases de type OXA-48, dans un échantillon biologique, comprenant les étapes consistant à :
a) mettre en contact l'échantillon biologique susceptible de contenir lesdites bactéries avec un milieu réactionnel comprenant un substrat chromogène, et de la témocilline à une concentration supérieure ou égale à 150 mg/L, de préférence comprise entre 200 et 500 mg/L,
   et un agent chélatant des cations divalents de type EDTA, préférentiellement à une concentration comprise entre 1,0 et 2,5 mmol/L,
b) incuber l'ensemble de façon à permettre la croissance des bactéries, et
c) détecter les souches correspondant aux bactéries productrices de carbapénémases de type OXA-48.

Avantageusement, le substrat chromogène permet de détecter une activité enzymatique et d'identifier les bactéries détectées.

De façon avantageuse, ledit milieu réactionnel est un milieu de culture. Le milieu réactionnel mis en oeuvre à l'étape a) comprend en outre un agent chélatant des cations divalents, de type EDTA, préférentiellement à une concentration comprise entre 1,0 et 2,5 mM.

Selon mode particulier de réalisation de l'invention, le milieu mis en oeuvre à l'étape a) comprend au moins un autre substrat chromogène permettant de détecter une activité enzymatique.

De façon préférentielle, l'activité enzymatique détectée pour permettre l'identification des bactéries est choisie parmi les activités estérase, glucosidase, galactosidase, et glucuronidase.

De façon préférentielle, les bactéries cibles susceptibles de produire des carbapénémases de type OXA-48 sont des entérobactéries.

Avantageusement, le procédé selon l'invention combine deux milieux réactionnels, l'un comprenant au moins un substrat chromogène, de la témocilline et un agent chélatant des catios divalents de type EDTA, l'autre comprenant au moins un substrat chromogène et un carbapénème, préférentiellement du faropénème. De façon préférentielle, une boite type boite de Petri bi-compartimentée, autrement appelée biboite, permet de combiner ces deux milieux. Une comparaison des colonies présentes après incubation permet d'identifier les souches correspondant aux bactéries résistantes aux carbapénèmes et/ou productrices de carbapénémases de type OXA-48.

La présente invention porte également sur un milieu de culture prêt-à l'emploi pour la détection et/ou l'identification spécifique d'entérobactéries productrices de carbapénémases de type OXA-48, comprenant :
- une base gélosée nutritive apte à la croissance des entérobactéries,
- de la témocilline à une concentration supérieure ou égale à 150 mg/L, de préférence comprise entre 200 et 500 mg/L,
- au moins un substrat chromogène, et un agent chélatant des cations divalents, de type EDTA, préférentiellement à une concentration comprise entre 1,0 et 2,5 mM.

Enfin, la présente invention est relative à l'utilisation de la témocilline à une concentration supérieure ou égale à 150 mg/L, préférentiellement à une concentration comprise entre 200 et 500 mg/L combiné à un agent chélatant des cations divalents de type EDTA, préférentiellement à une concentration comprise entre 1,0 et 2,5 mmol/L, dans un milieu réactionnel gélosé ou liquide, pour détecter et/ou identifier spécifiquement des bactéries productrices de carbapénémases de type OXA-48 susceptibles d'être comprises dans un milieu biologique.

Au sens de la présente invention, la témocilline est en phase homogène dans le milieu réactionnel, en présence de l'échantillon. La témocilline n'est pas imprégnée sur un disque ou sur une bandelette ou sur un autre contenant indépendant déposé sur ou dans le milieu réactionnel.

Les exemples développés ci-dessous ont pour but de faciliter la compréhension de l'invention. Ils sont donnés à titre explicatif et ne sauraient limiter la portée de l'invention.

### Exemple 1 : Détermination des CMI à la témocilline d'un panel de souches EPC (par la méthode de dilution en gélose)

Souchier utilisé :

| **Profil de résistance** | **Nb de souches** |
|---|---|
| Amp C | 10 |
| BLSE | 10 |
| IMP | 13 |
| KPC | 12 |
| NDM-1 | 26 |
| Résistance par imperméabilité (RI) | 10 |
| VIM | 16 |
| OXA-48 | 16 |

Milieu réactionnel : la base est un milieu chromID™ CPS (bioMérieux, ref. 43821-43829), comprenant un substrat chromogène de beta-glucuronidase, un substrat chromogène de beta-glucosidase, un substrat chromogène de beta-galactosidase et un substrat de désaminase. A ce milieu de base est ajoutée de la témocilline aux concentrations indiquées ci-après.
Gamme en Témocilline testée : 50 - 150 - 200 - 300 mg/L (Témocilline disodique, Eumedica SA, Manage, Belgique)

### Méthode

les milieux sont ensemencés par dépôt d'un spot : 1 µl d'une suspension microbienne en eau physiologique à 0.5 McF, diluée au 1/10.
Incubation 24 h à 37°C.

Lecture après 24 heures d'incubation. On retiendra comme CMI le milieu avec la plus petite concentration en témocilline sur lequel on obtiendra un spot de croissance négatif. Ont été considérés comme négatifs les spots ne présentant que 3 colonies ou moins ou une absence totale de croissance.

### Résultats

| **CMI** | **BLSE** | **KPC** | **NDM-1** | **OXA-48** | **RI*** | **AmpC** | **IMP** | **VIM** |
|---|---|---|---|---|---|---|---|---|
| **≤ 50** | 8 | 8 | 25 | 1 | 8 | 10 | 12 | 9 |
| **150** | 0 | 1 | 1 | 0 | 2 | 0 | 0 | 5 |
| **200** | 0 | 0 | 0 | 0 | 0 | 0 | 0 | 1 |
| **300** | 0 | 0 | 0 | 0 | 0 | 0 | 0 | 0 |
| **> 300** | 2 | 3 | 0 | 15 | 0 | 0 | 1 | 1 |

| | | | | | | | | |
|---|---|---|---|---|---|---|---|---|
| *RI : souches résistantes aux carbapénèmes par imperméabilité | | | | | | | | |

### Conclusion

La plupart des souches non-OXA-48 ont des CMI ≤ 200. La majeure partie des souches OXA-48 ont des CMI > 300. Seules quelques souches non OXA-48 ont également des CMI > 300.

Détection des souches productrices d'une carbapénémase de type OXA-48 (avec une concentration en Témocilline de 200 mg/L):

| | |
|---|---|
| Sensibilité | 93.8 % |
| Spécificité | 92.8 % |

La Témocilline semble donc être discriminante des souches productrices d'une carbapénémase de type OXA-48 dès la concentration 150 mg/L et très discriminante à 200 mg/L.

### Exemple 2 : Optimisation de la spécificité de détection par l'apport d'inhibiteurs spécifiques Inhibition des souches de classe B (NDM, VIM, IMP) par ajout d'EDTA.

Souchier utilisé :

| **Profil de résistance** | **Nb de souches** |
|---|---|
| AmpC | 10 |
| BLSE | 10 |
| IMP | 13 |
| KPC | 19 |
| NDM-1 | 27 |
| Résistance par imperméabilité (RI) | 10 |
| VIM | 17 |
| OXA-48 | 15 |

### Base peptonée :

Identique à celle utilisée pour déterminer les CMI à la Témocilline en dilution en gélose (Exemple 1)
Gamme en Témocilline 50-150-200-300 mg/l

Pour chaque concentration en Témocilline, on testera sous forme d' EDTA disodique, les concentrations en EDTA actif suivantes : 0, 1.77, 2.12, 2.47 et 2.83 mmol/L

Méthode : identique à la méthode mise en oeuvre à l'exemple 1.

### Résultats :

Répartition des CMI obtenues avec un milieu à 0 mmol/l d'EDTA

| **CMI** | **BLSE** | **KPC** | **NDM-1** | **OXA-48** | **RI** | **AmpC** | **IMP** | **VIM** |
|---|---|---|---|---|---|---|---|---|
| **≤ 50** | 10 | 18 | 22 | 0 | 8 | 10 | 12 | 9 |
| **150** | 0 | 0 | 4 | 0 | 2 | 0 | 0 | 6 |
| **200** | 0 | 0 | 1 | 0 | 0 | 0 | 0 | 1 |
| **300** | 0 | 0 | 0 | 3 | 0 | 0 | 0 | 0 |
| **> 300** | 0 | 1 | 0 | 12 | 0 | 0 | 1 | 1 |

Répartition des CMI obtenues avec un milieu à 1.77 mmol/l d'EDTA actif

| **CMI** | **BLSE** | **KPC** | **NDM-1** | **OXA-48** | **RI** | **AmpC** | **IMP** | **VIM** |
|---|---|---|---|---|---|---|---|---|
| **≤ 50** | 10 | 18 | 25 | 0 | 4 | 9 | 12 | 17 |
| **150** | 0 | 0 | 2 | 0 | 4 | 1 | 0 | 0 |
| **200** | 0 | 0 | 0 | 0 | 1 | 0 | 0 | 0 |
| **300** | 0 | 0 | 0 | 0 | 1 | 0 | 0 | 0 |
| **> 300** | 0 | 1 | 0 | 15 | 0 | 0 | 1 | 0 |

Répartition des CMI obtenues avec un milieu à 2.12 mmol/l d'EDTA actif

| **CMI** | **BLSE** | **KPC** | **NDM-1** | **OXA-48** | **RI** | **AmpC** | **IMP** | **VIM** |
|---|---|---|---|---|---|---|---|---|
| **≤ 50** | 9 | 18 | 26 | 0 | 4 | 9 | 12 | 17 |
| **150** | 1 | 0 | 1 | 0 | 3 | 1 | 0 | 0 |
| **200** | 0 | 0 | 0 | 0 | 2 | 0 | 0 | 0 |
| **300** | 0 | 0 | 0 | 0 | 1 | 0 | 0 | 0 |
| **> 300** | 0 | 1 | 0 | 15 | 0 | 0 | 1 | 0 |

Répartition des CMI obtenues avec un milieu à 2.47 mmol/l d'EDTA actif

| **CMI** | **BLSE** | **KPC** | **NDM-1** | **OXA-48** | **RI** | **AmpC** | **IMP** | **VIM** |
|---|---|---|---|---|---|---|---|---|
| **≤ 50** | 10 | 18 | 26 | 0 | 6 | 10 | 12 | 17 |
| **150** | 0 | 0 | 1 | 0 | 2 | 0 | 0 | 0 |
| **200** | 0 | 0 | 0 | 0 | 2 | 0 | 0 | 0 |
| **300** | 0 | 0 | 0 | 2 | 0 | 0 | 0 | 0 |
| **> 300** | 0 | 1 | 0 | 13 | 0 | 0 | 1 | 0 |

Répartition des CMI obtenues avec un milieu à 2.83 mmol/l d'EDTA actif

| **CMI** | **BLSE** | **KPC** | **NDM-1** | **OXA-48** | **RI** | **AmpC** | **IMP** | **VIM** |
|---|---|---|---|---|---|---|---|---|
| **≤ 50** | 10 | 19 | 27 | 3 | 6 | 10 | 12 | 17 |
| **150** | 0 | 0 | 0 | 1 | 3 | 0 | 0 | 0 |
| **200** | 0 | 0 | 0 | 0 | 1 | 0 | 0 | 0 |
| **300** | 0 | 0 | 0 | 3 | 0 | 0 | 0 | 0 |
| **> 300** | 0 | 0 | 0 | 8 | 0 | 0 | 1 | 0 |

Conclusion : les EPC de classe B de type VIM et NDM voient leur CMI diminuer en présence d'EDTA de manière proportionnelle à la concentration utilisée. Par contre, l'EDTA semble favoriser une légère augmentation des CMI pour les souches résistantes aux carbapénèmes par imperméabilité (RI).

Performances obtenues pour une concentration en Témocilline à 300 mg/L

| | Sensibilité en % | Spécificité en % |
|---|---|---|
| Milieu à 0 mmol/l EDTA | 100 | 97.2 |
| Milieu EDTA à 1.77 mmol/l | 100 | 98.1 |
| Milieu EDTA à 2.12 mmol/l | 100 | 98.1 |
| Milieu EDTA à 2.47 mmol/l | 86.7 | 98.1 |
| Milieu EDTA à 2.83 mmol/l | 53.3 | 99.1 |

L'apport d'EDTA permet une amélioration de spécificité, dès 1.77 mmol/L, sans dégradation de la sensibilité de détection des souches OXA-48 si la concentration reste <= 2.12 mmol/L.

### Exemple 3 : Evaluation des performances d'un milieu prototype défini.

Souchier utilisé :

| Profil de résistance | Nombre de souches |
|---|---|
| BLSE | 2 |
| IMP | 1 |
| KPC | 3 |
| NDM-1 | 1 |
| RI | 3 |
| VIM | 1 |
| OXA-48 | 15 |

### Méthode :

Les milieux sont préparés en boites de Petri diamètre 55 mm. Ils sont ensemencés à l'aide d'une suspension bactérienne en eau physiologique à 0.5 McF pour les souches non OXA-48 et avec une dilution au 1/100 et 1/10 000 d'une suspension à 0.5 McF pour les souches OXA-48. L'ensemencement consiste en un isolement 3 cadrans à l'aide d'un oëse calibrée de 10 µl : dépôt théorique de 10⁴ ou 10² CFU pour les souches OXA-48 et de 10⁶ CFU pour les souches non-productrices d'OXA-48.

Les milieux sont incubés à 37°C. Les lectures seront effectuées après 18 et 24 heures d'incubation.

La densité de croissance, les tailles de colonies, ainsi que la coloration (couleur et intensité) sont évaluées.

Milieu réactionnel identique à la base utilisée pour l'Exemple 1.Un système sélectif comprenant un mélange d'antibiotiques et d'antifongiques spécifique des Gram positifs, des levures et des Gram négatifs non fermentants, ainsi que de la cloxacilline et de l'EDTA, est également ajouté.
Gamme en Témocilline : 200, 300, 400, et 500 mg/L

Un milieu à 200 mg/L de témocilline est testé sans système sélectif tel qu'indiqué *supra.*

Résultats à 24 heures d'incubation:

| Concentration en Témocilline en mg/L | | | | | |
|---|---|---|---|---|---|
| Nb de souches détectées | 200 | 200 | 300 | 400 | 500 |
| | **sans système sélectif** | | | | |
| OXA-48 dépôt 10⁴ CFU | 15/15 | 15/15 | 15/15 | 15/15 | 12/15 |
| OXA-48 dépôt 10² CFU | 15/15 | 15/15 | 15/15 | 14*/15 | 9/15 |
| Non OXA-48 dépôt 10⁶ CFU | 3**/13 | 3**/13 | 1/13 | 2***/13 | 2***/13 |

| | | | | | |
|---|---|---|---|---|---|
| *dont 3 souches avec 1 seule colonie détectée ** dont 2 souches avec 1 seule colonie *** dont 1 souche avec1 seule colonie | | | | | |

Conclusion: l'ajout du système sélectif n'a pas d'impact sur la détection des souches OXA-48. La sensibilité tend à diminuer légèrement, dans cette configuration de milieu réactionnel, à partir de 400 mg/L de témocilline.

## Revendications

1. Procédé de détection et/ou d'identification spécifique de bactéries productrices de carbapénémases de type OXA-48 dans un échantillon biologique, comprenant les étapes consistant à :
a) mettre en contact l'échantillon biologique susceptible de contenir lesdites bactéries avec un milieu réactionnel comprenant au moins un substrat chromogène permettant de détecter une activité enzymatique, de la témocilline à une concentration supérieure ou égale à 150 mg/L, de préférence comprise entre 200 et 500 mg/L, et un agent chélatant des cations divalents de type EDTA, préférentiellement à une concentration comprise entre 1,0 et 2,5 mmol/L,
b) incuber l'ensemble de façon à permettre la croissance des bactéries, et
c) détecter les souches correspondant aux bactéries productrices de carbapénémases de type OXA-48.

2. Procédé selon la revendication 1, dans lequel les bactéries susceptibles d'être présentes dans l'échantillon sont des entérobactéries.

3. Procédé selon la revendication 2, dans lequel le milieu mis en oeuvre à l'étape a) comprend au moins un autre substrat chromogène permettant de détecter une activité enzymatique.

4. Procédé selon la revendication 2 ou 3, dans lequel le substrat permet de détecter au - moins une activité enzymatique choisie parmi les activités estérase, glucosidase, galactosidase, et glucuronidase.

5. Procédé de détection et/ou d'identification de bactéries productrices de carbapénémases de type OXA-48 et de bactéries résistantes aux carbapénèmes dans un échantillon biologique, comprenant les étapes consistant à :
d) mettre en contact l'échantillon biologique susceptible de contenir lesdites bactéries avec un milieu réactionnel comprenant au moins un substrat chromogène permettant de détecter une activité enzymatique et de la témocilline à une concentration supérieure à 150 mg/L, et un agent chélatant des cations divalents de type EDTA, préférentiellement à une concentration comprise entre 1,0 et 2,5 mmol/L,
et avec un autre milieu réactionnel comprenant au moins un substrat chromogène permettant de détecter une activité enzymatique et un carbapénème, préférentiellement du faropénème ;
e) incuber l'ensemble de façon à permettre la croissance des bactéries, et
f) détecter les souches correspondant aux bactéries résistantes aux carbapénèmes et/ou productrices de carbapénémases de type OXA-48.

6. Procédé selon la revendication 5 dans lequel l'étape a) est mise en oeuvre sur une biboite combinant deux milieux tels qu'indiqués à l'étape a).

7. Milieu de culture prêt à l'emploi pour la détection et/ou l'identification d'entérobactéries productrices de carbapénémases de type OXA-48, comprenant :
• une base gélosée nutritive apte à la croissance des entérobactéries,
• de la témocilline à une concentration supérieure ou égale à 150 mg/L, de préférence comprise entre 200 et 500 mg/L, et un agent chélatant des cations divalents, de type EDTA, préférentiellement à une concentration comprise entre 1,0 et 2,5 mmol/L,
• au moins un substrat chromogène.

8. Utilisation de la témocilline à une concentration supérieure ou égale à 150 mg/L, préférentiellement comprise entre 200 et 500 mg/L, combinée avec un agent chélatant des cations divalents, à une concentration préférentiellement comprise entre 1,0 et 2,5 mmol/L, dans un milieu réactionnel liquide ou gélosé, pour détecter et/ou identifier des bactéries productrices de carbapénémases de type OXA-48 susceptibles d'être comprises dans un échantillon biologique.

## Patentansprüche

1. Verfahren zum spezifischen Nachweis und/oder zur spezifischen Identifikation von Bakterien, die Carbapenemasen des OXA-48-Typs produzieren, in einer biologischen Probe, umfassend die folgenden Schritte:
a) Inkontaktbringen der biologischen Probe, die diese Bakterien enthalten kann, mit einem Reaktionsmedium, das mindestens ein chromogenes Substrat, das den Nachweis einer enzymatischen Aktivität gestattet, Temocillin in einer Konzentration von mehr als oder gleich 150 mg/l, vorzugsweise zwischen 200 und 500 mg/l, und ein Chelatbildungsmittel des EDTA-Typs für zweiwertige Kationen, vorzugsweise in einer Konzentration zwischen 1,0 und 2,5 mmol/l, umfasst,
b) Inkubieren des Gesamten, um das Wachstum der Bakterien zu ermöglichen, und
c) Nachweisen der Stämme, die Bakterien entsprechen, die Carbapenemasen des OXA-48-Typs produzieren.

2. Verfahren nach Anspruch 1, wobei die Bakterien, die in der Probe vorhanden sein können, Enterobakterien sind.

3. Verfahren nach Anspruch 2, wobei das in Schritt a) verwendete Medium mindestens ein anderes chromogenes Substrat umfasst, das den Nachweis einer enzymatischen Aktivität gestattet.

4. Verfahren nach Anspruch 2 oder 3, wobei das Substrat den Nachweis mindestens einer enzymatischen Aktivität gestattet, die aus Esterase-, Glucosidase-, Galactosidase- und Glucuronidase-Aktivitäten ausgewählt ist.

5. Verfahren zum Nachweis und/oder zur Identifikation von Bakterien, die Carbapenemasen des OXA-48-Typs produzieren, und von Bakterien, die gegenüber Carbapenemen resistent sind, in einer biologischen Probe, umfassend die folgenden Schritte:
d) Inkontaktbringen der biologischen Probe, die diese Bakterien enthalten kann, mit einem Reaktionsmedium, das mindestens ein chromogenes Substrat, das den Nachweis einer enzymatischen Aktivität gestattet, und Temocillin in einer Konzentration von mehr als 150 mg/l sowie ein Chelatbildungsmittel des EDTA-Typs für zweiwertige Kationen, vorzugsweise in einer Konzentration zwischen 1,0 und 2,5 mmol/l, umfasst,
und mit einem anderen Reaktionsmedium, das mindestens ein chromogenes Substrat, das den Nachweis einer enzymatischen Aktivität gestattet, und ein Carbapenem, vorzugsweise Faropenem, umfasst,
e) Inkubieren des Gesamten, um das Wachstum der Bakterien zu ermöglichen, und
f) Nachweisen der Stämme, die den gegenüber Carbapenemen resistenten Bakterien entsprechen und/oder die Carbapenemasen des OXA-48-Typs produzieren.

6. Verfahren nach Anspruch 5, wobei Schritt a) an einem Doppelbehälter durchgeführt wird, der zwei Medien, wie in Schritt a) angegeben, vereinigt.

7. Gebrauchsfertiges Kulturmedium zum Nachweis und/oder zur Identifikation von Enterobakterien, die Carbapenemasen des OXA-48-Typs produzieren, umfassend:
• eine für das Wachstum von Enterobakterien geeignete Nähragarbasis,
• Temocillin in einer Konzentration von mehr als oder gleich 150 mg/l, vorzugsweise zwischen 200 und 500 mg/l, und ein Chelatbildungsmittel des EDTA-Typs für zweiwertige Kationen, vorzugsweise in einer Konzentration zwischen 1,0 und 2,5 mmol/l,
• mindestens ein chromogenes Substrat.

8. Verwendung von Temocillin in einer Konzentration von mehr als oder gleich 150 mg/l, vorzugsweise zwischen 200 und 500 mg/l, in Kombination mit einem Chelatbildungsmittel für zweiwertige Kationen, vorzugsweise in einer Konzentration zwischen 1,0 und 2,5 mmol/1, in einem flüssigen oder Agar-Reaktionsmedium zum Nachweis und/oder zur Identifikation von Bakterien, die Carbapenemasen des OXA-48-Typs produzieren, die in einer biologischen Probe enthalten sein können.

## Claims

1. A method for specifically detecting and/or identifying OXA-48 carbapenemase-producing bacteria in a biological sample, comprising the steps consisting in:
a) bringing the biological sample which may contain said bacteria into contact with a reaction medium comprising at least one chromogenic substrate which makes it possible to detect an enzymatic activity, temocillin at a concentration greater than or equal to 150 mg/l, preferably between 200 and 500 mg/l, and a divalent-cation-chelating agent of EDTA type, preferentially at a concentration of between 1.0 and 2.5 mmol/L.
b) incubating the whole mixture so as to allow the bacteria to grow, and
c) detecting the strains corresponding to the OXA-48 carbapenemase-producing bacteria.

2. The method as claimed in claim 1, wherein the bacteria which may be present in the sample are Enterobacteriaceae.

3. The method as claimed in claim 2, wherein the medium used in step a) comprises at least one other chromogenic substrate which makes it possible to detect an enzymatic activity.

4. The method as claimed in claims 2 or 3, wherein the substrate makes it possible to detect at least one enzymatic activity chosen from esterase, glucosidase, galactosidase and glucuronidase activities.

5. A method for detecting and/or identifying OXA-48 carbapenemase-producing bacteria and carbapenem-resistant bacteria in a biological sample, comprising the steps consisting in:
a) bringing the biological sample which may contain said bacteria into contact with a reaction medium comprising at least one chromogenic substrate which makes it possible to detect an enzymatic activity, temocillin at a concentration greater than 150 mg/l, and a divalent-cation-chelating agent of EDTA type, preferentially at a concentration of between 1.0 and 2.5 mmol/L.
and with another reaction medium comprising at least one chromogenic substrate which makes it possible to detect an enzymatic activity and a carbapenem, preferentially faropenem;
b) incubating the whole mixture so as to allow the bacteria to grow, and
c) detecting the strains corresponding to the carbapenem-resistant and/or OXA-48 carbapenemase-producing bacteria.

6. The method as claimed in claim 5, wherein step a) is carried out on a biplate combining two media as indicated in step a).

7. A ready-to-use culture medium for the detection and/or identification of OXA-48 carbapenemase-producing Enterobacteriaceae, comprising:
• a nutritive agar base suitable for the growth of Enterobacteriaceae,
• temocillin at a concentration greater than or equal to 150 mg/l, preferably between 200 and 500 mg/l, and a divalent-cation-chelating agent of EDTA type, preferentially at a concentration of between 1.0 and 2.5 mmol/L.
• at least one chromogenic substrate.

8. A use of temocillin at a concentration greater than or equal to 150 mg/l, preferentially between 200 and 500 mg/l, combined with a divalent-cation-chelating agent of EDTA type, preferentially at a concentration of between 1.0 and 2.5 mmol/L, in a liquid or agar reaction medium, for detecting and/or identifying OXA-48 carbapenemase-producing bacteria which may be included in a biological medium.
